Europäisches Patentamt

European Patent Office

Office européen des brevets

(11) Publication number: **0 499 932 A1**

# EUROPEAN PATENT APPLICATION

(21) Application number: **92102162.2**

(22) Date of filing: **10.02.92**

(51) Int. Cl.5: **C12N 5/00**

(30) Priority: **16.02.91 JP 42449/91**
       **19.09.91 JP 268233/91**

(43) Date of publication of application:
**26.08.92 Bulletin 92/35**

(84) Designated Contracting States:
**DE ES FR GB IT SE**

(71) Applicant: **W.R. Grace & Co.-Conn. (a Connecticut corp.)**
**Grace Plaza 1114 Avenue of the Americas**
**New York New York 10036(US)**
Applicant: **Kubota, Sunao**
**21-24 Higashi 3-chome**

**Kunitachi-shi, Tokyo-to(JP)**

(72) Inventor: **Mori, Yuichi**
**1642-212-B-4 Kamariva-cho, Kanazawa-ku**
**Yokohama-shi, Kanagawa-ken(JP)**
Inventor: **Takezawa, Toshiaki**
**Nifty 34-302, 31-15 Higashinaruse**
**Isehara-shi(JP)**

(74) Representative: **UEXKÜLL & STOLBERG**
**Patentanwälte**
**Beselerstrasse 4**
**W-2000 Hamburg 52(DE)**

(54) **Cancer cell cluster comprising cancer cells and normal cells and preparation thereof.**

(57) A cancer cell cluster comprising cancer cells and normal cells, preferably primary cancer cells isolated directly from a patient tumor, and having a controlled size and a controlled composition of both the cancer cells and the normal cells is disclosed. A method for preparing the same involves (i) seeding and culturing normal cells on a cell culture substrate comprising (a) a temperature-responsive polymeric compound having an LCST and (b) a cell adhesive substance at a temperature higher than said LCST until said normal cells form a confluent monolayer of normal cells; (ii) seeding and attaching cancer cells on said monolayer of normal cells at a temperature higher than said LCST; (iii) detaching the monolayer of normal cells attached with said cancer cells from said cell culture substrate by lowering said temperature to a temperature below said LCST; and (iv) culturing the detached monolayer on a cell non-adhesive substrate to form a cancer cell cluster.

FIG. 1

EP 0 499 932 A1

## Technical Field

The present invention relates to a cancer cell cluster comprising cancer cells and normal cells. More specifically, it relates to a cancer cell cluster comprising cancer cells and normal cells and having a controlled size and a controlled composition of the cancer cells and the normal cells. The present invention also relates to a method for preparing the cancer cell cluster comprising cancer cells and normal cells.

## Background Art

Culture systems of cancer cells have been used to evaluate the efficacy of chemotherapy or physical therapy, such as radiation and hyperthermia. The culture systems developed so far can be categorized into the following three types; (1) a two dimensional culture system, (2) a culture system in gel and (3) an aggregated cell culture system. It is important that the culture systems are capable of culturing primary cancer cells removed directly from a patient. Further, the culture systems should maintain the original characteristic features of the patient's tumors for a long period of time. The original characteristic features of an *in vivo* tumor include not only the functions of the primary cancer cells themselves, but also the microregional architecture surrounding the *in vivo* tumor such as the arrangement of normal cells, interstitial tissue and blood vessels. However, according to the above-described conventional culture systems it has hardly been possible to construct cultured cancer cell systems having such a similarity to *in vivo* tumors. Accordingly, it has been very difficult to extrapolate the clinical efficacy of a therapy by measuring the effects which the therapy exerts on conventional cultured cancer cell systems.

From the viewpoint of the similarity to *in vivo* tumors, the problems associated with the above described conventional culture systems may be summerized below.

(1) The two-dimensional culture system has been most widely used for cancer cell culture. This system usually cultures established cancer cell lines, but it is the least appropriate for the culture of primary cancer cells directly isolated from patient's tumors. This is a serious disadvantage of the two dimensional culture system, since most of the established cancer cell lines are considered to lose the original characteristic features of the primary cancer cells in their establishing processes. The reason why the two dimensional culture system is not suitable for the primary cancer cell culture is that fibroblasts very often contaminate the primary cancer cells during the isolation process from patient's tumors and strongly impede the growth of the primary cancer cells, because fibroblast is a cell which shows the most potent growth activity in the two dimensional culture system. It is very difficult to isolate only primary cancer cells from patient's tumors without any contamination of fibroblasts.

To address this problem, a two-dimensional culture which can comparatively suppress the growth of fibroblasts has been developed. As a result, methods have been developed for the culturing and proliferation of primary cancer cells on a monolayer (cell mat) of Balb/3T3 cell derived from mice, which method can prevent contact of fibroblasts [K. Miki, et al, Tissue Culture Research Communications (1991) 9:35], and for the proliferation of only the lung cancer cells on a monolayer (cell mat) of C3H10T1/2 cell line to hinder the growth of the normal cells in the lung tumor [Klein, J.C. et al, Cancer Res. (1987) 47:3851]. However, even if the growth of the coexisting fibroblasts is suppressed by these methods, the micro environment of the two dimensional culture system is significantly different from that surrounding an *in vivo* tumor. This is another crucial problem of the two dimensional cell culture system. According to the two dimensional culture, the architecture of a cultured cell cluster is plane and morphologically different from the three dimensional structure of *in vivo* tumors because the cultured cell cluster is firmly backed with the plane substrate. As a typical example of the morphological difference, the evidence that while adenocarcinoma usually forms a glandular structure *in vivo*, no formation of such a structure is recognized in the conventional two dimensional culture. This is because the plane structure firmly backed with a substrate impedes the formation of the three dimensional glandular structure.

(2) The cell culture system in gel is a typical three dimensional culture. It has been reported that three-dimensional culture is superior to two-dimensional culture in maintaining the original characteristic features of cancer cells for a longer period of time [e.g. Miller, B.E. et al, Cancer Res. (1985) 45:4200]. For the gel culture systems, a method of culturing cancer cells using collagen gel as the matrix [J. Enami et al, Tissue Culture Research Communications (1983) 9:397], and a method of culturing cancer cells in double soft agar media [N. Tanigawa et al, Cancer Res. (1982) 42:2159] have been developed. The major problem of the gel culture system is that it is very difficult to sufficiently supply nutrients into the inside of the gel and also to effectively remove accumulated wastes from the gel. This problem not only impedes cell growth but also causes heavy necrosis, and accordingly makes the culture of delicate

2

primary cancer cells difficult.

(3) The aggregated cell culture system is considered most suitable for the formation of a cancer cell cluster similar to *in vivo* tumors, because the morphology of the aggregated cancer cells is very close to that of *in vivo* tumors. The aggregated cell clusters are formed by spontaneous aggregation of cancer cells in spin culture or by spontaneous detachment of cancer cell colonies which are weakly attached onto a substrate coated with non-adhesive materials such as agar [J. Calsson, et al, Spheroids in Cancer Research (1984), 1, edited by Acker, H., et al, Springer-Verlag]. The cancer cell clusters prepared by these methods morphologically resemble the *in vivo* solid tumor. The cell clusters of established cancer cell lines prepared by these methods have been used as a model of solid tumors for the evaluation of chemosensitivity and radiosensitivity [R. M. Sutherland, Science (1988), 240:177]. However, using these methods it is almost impossible to control the size of the cancer cell cluster, since the spontaneous aggregation or detachment which makes the cell cluster, occurs accidentally and unexpectedly. In addition, a mass production of the cancer cell cluster having a desired size is hardly possible for the same reason. Since the size of the cell cluster is a very important factor to simulate chemosensitivity or radiosensitivity of the actual tumor, incapability of controlling the size is one of the crucial problems of the conventional aggregated cell culture method.

On the other hand, another problem of this method is that it is very difficult to control the composition of cancer cells and normal cells. In actual tumors, normal cells coexist and interact with cancer cells. Therefore, the control of the composition of both cancer cells and normal cells is actually necessary to more effectively simulate *in vivo* tumors. Particularly, the coexistence of normal cells is imperative for the estimation of the side effects of the therapy. This is because the side effects would be closely correlated to the effects which the therapy exerts on the normal cells in the cluster. According to this method, the incapability of controlling the cell composition of the cluster is induced by the difference in aggregability of cancer cells and normal cells. Generally, the aggregability of cancer cells is much higher than that of normal cells. Using this method the preparation of a cell cluster comprising fibroblasts as normal cells and HeLa cells as a cancer cell line has been attempted [Sasaki, T., Cancer Res. (1985) 34:21]. As a result, it has been noticed that formation of the cluster of cancer cells having a low aggregating tendency could be improved by coculturing the cancer cells with fibroblasts. However, in this coculture system, the aggregability of the cancer cell line is significantly higher than that of the fibroblast and as a result, practically the mixed cell cluster was formed only from the cancer cell line. Therefore, with this method the composition ratio of cancer cells to fibroblast cells in the mixed cell cluster cannot be controlled and also it is difficult to obtain the cell cluster in a high yield.

On the other hand, attempts have been made to form cancer cell clusters from primary cancer cells directly isolated from a patient instead of cancer cell lines by using the above described method [J. L. Darling, et al, Cell Biology International Reports (1983) 7:23, and E. K. Rofstad, Br. J. Cancer (1986) 54:569]. Formation of a cell cluster using primary cancer cells directly isolated from individual patient has been attempted, but its rate of success has been significantly lower than using an established cancer cell line. In this method, formation of the cell cluster is based on the use of the spontaneous aggregability of the cancer cell. Therefore, the low success rate may be attributed to the fact that the aggregability of primary cancer cells significantly differs depending on the species of primary cancer cells and thus, the method cannot be utilized for all the species of primary cancer cells. Perhaps, the aggregability of primary cancer cells isolated directly from a patient would scatter much more widely than that of established cell lines.

As briefly described above, it is difficult to culture or grow primary cancer cells isolated directly from patients by the culture techniques of the prior art. Furthermore, it is extremely difficult to prepare a primary cancer cell cluster mixed with a constant amount of normal cells, even though such a cluster is required to measure its influence on the normal cell for the evaluation of the side effect of chemotherapy or physical therapy *in vivo*. It is also very difficult to control the size of a cancer cell cluster or to improve its yield, and these are the important conditions required for the quantitative measurement of the chemosensitivity or radiosensitivity. Thus, the cancer cell cluster of the prior art and its preparation method have the above described serious defects.

The present invention provides a cancer cell cluster comprising cancer cells, especially primary cancer cells isolated directly from a patient's tumor, and normal cells having a controlled size and a controlled composition of both the cancer cells and the normal cells. The cluster is useful for the selection of a most effective therapeutic condition for a cancer patient, more specifically chemotherapeutic conditions such as species and dosage of an anti-cancer drug and the conditions of physical therapy such as radiation and hyperthermia as well as for the evaluation of side effects of the above described therapies by measuring the effect exerted on the normal cells in the cancer cell cluster. The present invention also provides a method for preparing such a cancer cell cluster.

**Definitions**

The term "cancer cell" is used herein to mean a transformed cell, more definitely, an animal cell which shows chromosome abnormality by G-Band nucleo analysis [M. E. Drets, et al, Proc. Natl. Acad. Sci., U.S.A. (1971) 68:2073]. In the present invention contact inhibition can be used as a measure of the transformed cell, since cancer cell generally shows no contact inhibition. The contact inhibition of a cell can be judged by whether the cell proliferates or not after a confluent stage in a monolayer culture.

The term "primary cancer cell" is used herein to mean cancer cells isolated directly from a patient's tumor, which is diagnosed by localization of cell nucleas, size and deep stainability or the presence or the absence of adenotube structure.

The term "normal cell" is used herein to mean an animal cell excluding the above described cancer cell. In other words, it refers to the animal cell which is not in a state of malignant transformation. In the present invention, fibroblast which exist in abundance *in vivo* and can be relatively easily isolated is the most preferred normal cell. If desired or necessary, two or more kinds of normal cells can be used. Sometimes it is possible to better simulate the surrounding structure of *in vivo* tumors by using two or more kinds of normal cells.

The term "LCST" is used herein to mean a lower critical solution temperature of a temperature-responsive polymeric compound between hydration and dehydration.

**SUMMARY OF THE INVENTION**

The cancer cell cluster in accordance with the present invention comprises cancer cells, especially primary cancer cells isolated directly from patient's tumors, and normal cells and has a controlled size and a controlled composition of the both cell species.

In another aspect of the present invention, a method for preparing a cancer cell cluster comprising cancer cells, especially primary cancer cells isolated directly from patient's tumors, and normal cells comprises the steps of:

(i) seeding and culturing normal cells on a cell culture substrate comprising,

(a) a temperature-responsive polymeric compound having an LCST lower than the culture temperature and

(b) a cell adhesive substance, at a temperature higher than said LCST until said normal cells form a confluent monolayer of normal cells;

(ii) seeding and attaching cancer cells on said monolayer of normal cells at a temperature higher than said LCST;

(iii) detaching the monolayer of normal cells attached with said cancer cells from said cell culture substrate by lowering said temperature to a temperature below said LCST; and

(iv) culturing the detached monolayer on a cell non-adhesive substrate to form a cancer cell cluster.

**BRIEF DESCRIPTION OF THE DRAWINGS**

FIG. 1 is a photograph showing one embodiment of the cancer cell cluster of the present invention. The cancer cell cluster consisting of fibroblasts derived from human skin and primary cancer cells isolated directly from a patient with stomach cancer and is stained with hematoxylin-eosin after culturing for 21 days. The cancer cell is indicated by an arrow in the photograph.

FIG. 2 is a photograph showing the original stomach tumor shown in FIG. 1. The cancer cell is indicated by an arrow in the photograph.

FIG. 3 is a photograph showing another embodiment of the cancer cell cluster of the present invention. The cancer cell cluster consists of fibroblasts derived from human skin and a cancer cell line established from cholangiocarcinoma and is stained with hematoxylin-eosin after culturing for 7 days. A number of pseudoglandular structures can clearly be observed in the cancer cell cluster.

FIG. 4 is another photograph of the cancer cell cluster of FIG. 3. The cancer cell cluster is stained with Carbohydrate Antigen 19-9. The cancer cells are positively stained and form the pseudoglandular structure.

FIG. 5 is a further photograph of the cancer cell cluster of FIG. 3. The cancer cell cluster is stained with Carcino Embryonic Antigen. The cancer cells surrounding the pseudoglandular structure are positively stained.

FIG. 6(a) is a photograph of the original cholangiocarcinoma of a patient (E. K.). The cancer cells are stained with hematoxylin-eosin. The photograph clearly shows the original glandular structure which morphologically resembles the pseudoglandular structure of the cancer cell cluster of FIG. 3.

FIG. 6(b) is a schematic drawing of the glandular structure of FIG. 6(a). Numerals 1 and 2 show a wall of the pseudoglandular structure and a lumen of the pseudoglandular structure, respectively.

## DETAILED DESCRIPTION OF THE INVENTION

A cell cluster comprising cancer cells and normal cells having a controlled size and a controlled composition of both the cell species can be prepared as described below.

Fibroblasts maintained at a temperature above the LCST of a temperature-responsive polymeric compound, for example, at 37°C are inoculated onto the bottom of a dish which has been coated with a mixture of collagen and the temperature-responsive polymeric compound, e.g., poly(n-isopropyl acrylamide) (herein "PNIPAAm"), and cultured at a temperature above the LCST until the bottom of the dish is fully covered by the fibroblasts in a confluent state.

Then, a suspension of cancer cells, especially, primary cancer cells isolated directly from a patient's tumor, maintained at a temperature above the LCST, for example, at 37°C is seeded on the confluent monolayer of fibroblasts so that the cancer cells can be completely attached onto the fibroblast monolayer. The cancer cell-attached fibroblast monolayer is detached from the bottom of the dish and is suspended in the culture medium as a cell sheet when the temperature of the dish is lowered to a temperature below the LCST, for example, at room temperature; about 25°C. If desired or necessary, the cell sheet may be washed two or three times with a phosphate buffer solution, and then the cell sheet is transferred to a cell non-adhesive dish and cultured until a cell cluster is formed.

In the above process, the size of the cell cluster and the cell composition depend on the number of normal cells, such as fibroblasts, and the number of cancer cells in the cell cluster. The number of fibroblasts in the cluster can be calculated by multiplying the bottom area of the culture dish by the cell density in the confluent monolayer. The cell density of the monolayer can easily and precisely be calculated by conventional methods such as phase contrast microscopic observation before seeding the cancer cells. The cell density of the confluent monolayer depends on the species of cells. For example, in the case of fibroblasts, the cell density of the confluent state is $6 \sim 8 \times 10^4$ cells/cm$^2$. Therefore, the number of fibroblasts in the cell cluster is governed solely by the culture area of the dish. On the other hand, the number of cancer cells attached on the fibroblast monolayer can be measured directly by the phase contrast microscopic observation or by subtracting the number of non-attached cancer cells still suspended in the culture medium from the number of the cancer cells seeded. Thus, the number of cancer cells in the cell cluster can be controlled by the concentration of the cancer cells inoculated on the monolayer of fibroblasts. However, unlike the normal cells such as fibroblast, the cancer cells may proliferate within the cell cluster. Therefore, there is a possibility that the number of cancer cells may vary with time. In the initial stage of formation of said cell cluster, the composition and the size of the cell cluster of the present invention can be controlled.

The method for preparing a cultured cancer cell cluster is not particularly limited to any specific method. However, use of a temperature-responsive polymeric compound is preferred from the viewpoint of effectively preventing unnecessary damage to the cells.

The term "temperature-responsive polymeric compound" refers to a polymer having an LCST (lower critical solution temperature) [For example, Heskins, M., et al, J. Macromol. Sci-Chem (1968) A2 8: 1441]. In the present invention, the temperature-responsive polymeric compound with an LCST of 0 to 80°C, preferably 10 to 40°C is typically employed. For example, a temperature-responsive polymeric compound is dissolved in a certain aqueous solution such as a culture medium at a temperature below its LCST. Then the temperature is raised at a rate of about 3°C per minute by using a melting point measuring device while observing the temperature by the naked eyes to see where turbidity starts to form. The temperature at which turbidity starts to form is the LCST of the temperature-responsive polymer [For details about the LCST, see T. Takezawa et al., Bio/Technology (1990), 8:654].

Preferred temperature-responsive polymeric compounds which can be used in the present invention are in a solid state at a temperature above their LCSTs and reversibly become water soluble when the temperature is lowered to a temperature below the LCST. Cells can be attached to grow on the temperature-responsive polymeric compound as a culture substrate, because the polymeric compound is in a solid state at a temperature higher than the LCST.

On the other hand, when it is necessary to detach proliferated cells, they can easily be removed without damaging the junctions among cells by lowering the temperature to a temperature below the LCST, because the temperature-responsive polymeric compound will be dissolved in the culture medium at a temperature below the LCST.

Examples of such temperature-responsive polymeric compounds include poly-N-substituted acrylamide or methacrylamide derivatives and their copolymers, polyvinylmethyl ether, polyethylene oxide, etherized methylcellulose and partially acetylated polyvinyl alcohol. Particularly preferred are poly-N-substituted methacrylamide or acrylamide derivatives and their copolymers, polyvinylmethyl ether and partially acetylated polyvinyl alcohol.

Preferred examples of such temperature-responsive polymeric compounds are listed below. The LCSTs of these polymers rise with the sequence of polymers listed below.

Poly(N-acryloyl piperidine);
Poly(N-n-propyl methacrylamide);
Poly(N-isopropyl acrylamide);
Poly(N,N-diethyl acrylamide);
Poly(N-isopropyl methacrylamide);
Poly(N-cyclopropyl acrylamide);
Poly(N-acryloyl pyrrolidine);
Poly(N,N-ethylmethyl acrylamide);
Poly(N-cyclopropyl methacrylamide);
Poly(N-ethyl acrylamide).

The above described polymers may be homopolymers or copolymers with other monomers. Any hydrophilic or hydrophobic monomers can be used as the monomers for copolymerization. Generally speaking, copolymerization with a hydrophilic monomer will raise the LCST, and copolymerization with a hydrophobic monomer will lower the LCST. With an appropriate selection of the monomers, a copolymer having a desired LCST can be obtained.

Examples of suitable hydrophilic monomers are N-vinyl-pyrrolidone, vinylpyridine, acrylamide, methacrylamide, N-methyl acrylamide, hydroxyethyl methacrylate, hydroxyethyl acrylate, hydroxymethyl methacrylate, hydroxymethyl acrylate, acrylic acid and methacrylic acid and their salts, vinylsulfonic acid and styrylsulfonic acid and their salts, and N,N-dimethylaminoethyl methacrylate, N,N-diethylaminoethyl methacrylate, and N,N-dimethylaminopropyl acrylamide and their salts, but the present invention is not limited to these compounds.

Since the cell membrane is normally negatively charged, it is preferred to use a copolymer of a monomer capable of giving a temperature-responsive polymeric compound and a monomer having a basic group to improve the attachability of cells on a substrate by electrostatic interaction.

Examples of suitable hydrophobic monomers are acrylate derivatives and methacrylate derivatives such as ethyl acrylate, methyl methacrylate and glycidyl methacrylate; N-substituted alkyl acrylamide derivatives and N-substituted alkyl methacrylamide derivatives such as N-n-butyl acrylamide and N-n-butyl methacrylamide; vinyl chloride; acrylonitrile; styrene; and vinyl acetate but the present invention is not limited to these compounds.

It is preferred that the molecular weight of the temperature-responsive polymeric compound which can be used in the present invention is at least $1.0 \times 10^5$. More preferred are molecular weights of at least $1.0 \times 10^6$. The molecular weight herein means a number average molecular weight determined from the viscosity. For example, the relationship between the number average molecular weight ($\overline{M}n$) of poly(N-isopropyl acrylamide) and its intrinsic viscosity [$\eta$] can be represented by the following equation [Ito, S. and Geronimo, R.T., Sen'i Kobunshi Zairyo Kenkyusho Hokoku (1988) 159:23];

$$[\eta] = 9.59 \times 10^{-5} \overline{M}n^{0.65}$$
(in tetrahydrofuran solution at 27°C)

Use of the temperature-responsive polymeric compound alone as a culture substrate lacks affinity for cells and cells cannot attach or grow. In order to improve the attachability and proliferation of cells on the culture substrate, the following cell adhesive substances are used together with the temperature-responsive polymeric compound. Examples of such cell adhesive substances which can be used in the present invention include collagen, fibronectin, vitronectin, laminin, proteoglycan, glycosaminoglycan, thrombospondin, gelatin, lectin, adhesive proteins isolated from shellfish, anchorage oligopeptides and positively charged polymers.

Examples of such positively charged polymers include polylysin, polyhistidine, protamine sulfate, polydimethylaminoethyl acrylate or methacrylate, polydiethylaminoethyl acrylate or methacrylate, polydimethylaminopropyl acrylate or methacrylate, polyethyleneimine and polyvinylpyridine.

The cell culture substrate which can be used in the present invention typically comprises a coating of a temperature-responsive polymeric compound having an LCST lower than the cell culture temperature and a cell adhesive substance formed on a supporting material.

6

Such a substrate can be prepared by coating an aqueous solution of a mixture of the temperature-responsive polymeric compound and the cell adhesive substance on all or part of the supporting material at a temperature below the LCST and drying the coating thus obtained. The substrate of the present invention can also be prepared by dipping the supporting material in an aqueous solution of a mixture of the temperature-responsive polymeric compound and the cell adhesive substance at a temperature below LCST and then drying the coating thus obtained. Further, the substrate used in the present invention can be prepared by forming a layer of the temperature-responsive polymeric compound on a supporting material, and then forming a layer of the cell adhesive substance on the layer of the temperature-responsive polymeric compound.

The mixing weight ratio of the temperature-responsive polymeric compound to the cell adhesive substance is typically from about 1:0.01 to about 1:3, and this ratio may vary depending on the type of the cell adhesive substance used and the cell species used.

Thickness of the coating after it is dried is at least about 0.2 $\mu$m, preferably at least about 0.5 $\mu$m and more preferably at least about 1.0 $\mu$m. When the thickness is below about 0.2 $\mu$m, the cell detachability remarkably worsens and it takes a very long time for the detachment of the cell and as a result, the cell functions are rendered unstable.

The supporting material which can be used in the present invention is preferably transparent or translucent, and preferably of glass or of plastic. Exemplary plastics include polystyrene, polyester, polyamide, polyethylene, polypropylene, polycarbonate, polymethyl methacrylate, polyoxymethylene, polyvinyl chloride, polyacrylonitrile, polyvinylidene fluoride, polytetrafluoroethylene and polydimethylsiloxane.

There is no particular limitation on the shape of the supporting material, and it can take various shapes such as a dish, plate, film or sheet.

The method for preparing a cancer cell cluster of the present invention will now be described. First, normal cells are cultured on a cell culture substrate comprising a temperature-responsive polymeric compound, and a cell adhesive substance at a temperature above the LCST of the temperature-responsive polymeric compound, typically at a temperature 1 to 40°C above the LCST, preferably at a temperature 3 to 20°C above the LCST until a confluent monolayer of the normal cells is formed on the cell culture substrate. Second, cancer cells, especially primary cancer cells isolated directly from a patient, are seeded and attached on the monolayer of the normal cells at a temperature above the LCST. Third, the cancer cell-attached monolayer of the normal cells is detached from the cell culture substrate by lowering the temperature to a temperature below the LCST, typically 1 to 40°C below the LCST preferably 3 to 20°C below the LCST to dissolve the temperature-responsive polymeric compound in the culture medium. Fourth, the cancer cell-attached monolayer of the normal cells is transferred onto a cell non-adhesive substrate and cultured to give a cancer cell cluster. As described above, one of the important characteristic features of the present invention is the preparation of a cancer cell cluster which retains the original functions of the cancer cell, especially primary cancer cell, by freeing and liberating the cells from the two dimensional culture system which is unsuitable for cancer cell culture. Furthermore, by using the temperature-responsive polymeric compound of the present invention, conventional cell detaching agents, such as proteolytic enzymes and calcium chelating agents for releasing or liberating cells from the cell culture substrate can be avoided. Thus, there is no chance for the cell monolayer to disintegrate into pieces, and at the same time, the damage to various receptors existing on the cell surface can be drastically minimized. As a result, the functions of a cancer cell, especially a primary cancer cell can be advantageously maintained. Furthermore, the presence of both cancer cells and normal cells in the cell cluster permits the simultaneous evaluation of effects of an anti-cancer agent on the cancer cells and the side effect of the anti-cancer agent on the normal cells under the same condition. Furthermore, according to the present invention the mixing ratio of the cancer cells to the normal cells in the culture of the cancer cell cluster can easily be controlled in its preparation steps, such as by regulating the number of both the cancer cells and normal cells at the time of seeding and attaching the cancer cells on a confluent monolayer of the normal cells.

The examples which follow are given for illustrative purposes and not meant to limit the present invention.

Example 1

A tumor (0.5 cm x 0.5 cm x 0.3 cm) was isolated from a gastric cancer patient (T. K., female, 38 years old) under an aseptic condition and immersed in a Hanks solution containing penicillin (100 U/ml), gentamycin (50 $\mu$g/ml), kanamycin (100 $\mu$g/ml) and fungizone (0.5 $\mu$g/ml) for about 30 minutes. It was then placed in a Petri dish and cut into thin strips of 1 to 3 mm$^3$. Then, the cancer tissue section was digested in a RPMI-1640 culture solution (TIL) containing 0.01% hyaluronidase, 0.004% DNAase, and 0.1% collagenase (type V) at 37°C by stirring with a stirrer for about 60 minutes. The cancer cells were isolated by filtering the digested solution through a 40 $\mu$m mesh, carrying out specific gravity centrifugation at 2000 r.p.m. for 20 minutes using Ficol and removing the blood components. The cancer cells were then suspended in DMEM media to form a concentration of about $2.0 \times 10^4$ cells/ml and $1.0 \times 10^5$ cells/ml, respectively.

A typical temperature-responsive polymeric compound, PNIPAAm having a number average molecular weight of $1 \times 10^6$ and an LCST of 28.6°C was dissolved in distilled water to prepare an aqueous solution (0.5% w/v) at 10°C, and sterilized by filtering through a sterile filter. The polymeric compound solution was mixed with an equal amount of an aqueous pepsin-digested type I collagen solution from cow's skin (0.5% w/v) at 10°C to form a mixed solution of PNIPAAm and collagen. About 160 $\mu$l and 180 $\mu$l of this mixed solution were injected into the bottom area of a 12 well Cultureware (the bottom area of the dish: about 3.8 cm², a product of Iwaki Glass K.K.) and the bottom area of a 4 well Multidish (the bottom area of the dish: about 1.9 cm², a product of Intermed-Japan K.K.), respectively, and air-dried at 10°C aseptically in a clean hood. Thus a dish with the bottom area coated with 2 $\mu$m thickness of the collagen and PNIPAAm mixture (weight ratio 1:1) was obtained.

A suspension of fibroblasts derived from human skin in the DMEM media (containing 10% fetal calf serum) was kept at 37°C and injected into the dish coated with the above described collagen/PNIPAAm mixture kept at 37°C. The seeding concentration of fibroblasts was about $4 \times 10^4$ cell/cm². The fibroblasts were cultured in a 37°C carbon dioxide incubator (air/5% carbon dioxide) for 5 days. As a result, the bottom area of the dish was completely covered with fibroblasts. Then, 0.5 ml of the DMEM suspension of the above mentioned cancer cells (cell concentrations: about $2.0 \times 10^4$ cells/ml; and $1.0 \times 10^5$ cells/ml) was maintained at 37°C and then injected to the above obtained dish having a fibroblast monolayer and left to stand at 37°C in a carbon dioxide incubator (air/5% carbon dioxide) for 17 hours. As a result, the above described cancer cell was attached on the confluent fibroblast monolayer. Then, the dish was removed from the 37°C incubator and cooled down to 4°C. As a result, the cancer cell-attached monolayer of fibroblasts was completely detached from the bottom of the dish as a cell sheet. When the detaching process of this monolayer was observed under a phase contrast microscope, it was found that the cell layer was gradually detached from the edge of the dish toward the center, and it was also confirmed that the detached monolayer of the fibroblasts wrapped up the attached cancer cells in an engulfing fashion. Separation of the cancer cells from the monolayer of the fibroblasts was not observed during the detaching process of the cell sheet. The detachment of the cell sheet was completed in about 5 minutes and the detached cell sheet was suspended in the culture medium. The cell sheet was washed with a phosphate buffer solution 2 to 3 times to remove dissolved PNIPAAm and collagen. Then, the sheet was transferred to a cell non-adhesive dish, i.e., a dish coated with agar and cultured in a 37°C carbon dioxide incubater (air/5% carbon dioxide). During the suspension culture, the cell sheet was transformed into a cell cluster in the cell non-adhesive dish.

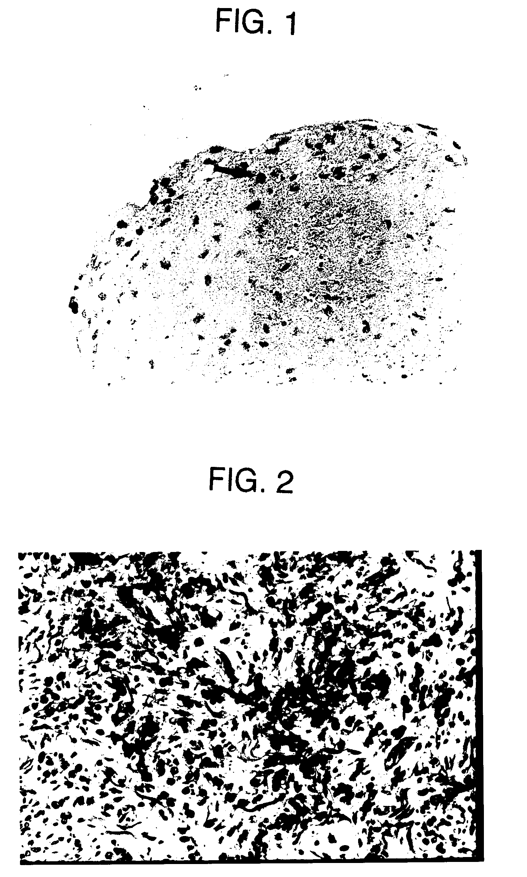

On the second day of suspension culture, the cultured cancer cell cluster was fixed by means of a 10% formalin buffer solution and a paraffin-embedded tissue section was prepared. The cancer cell cluster section was stained with hematoxylin-eosin, and the composition ratio of the cancer cell to the fibroblast and the size of the cluster were measured under a microscope. The results are shown in Table 1. After culturing the suspension for 21 days, the cluster was fixed and the tissue section was prepared in the same manner as described above. The result of staining the cancer cell section with hematoxylin-eosin is shown in FIG. 1.

For comparison, hematoxylin-eosin staining of the original tumor of a cancer patient (T.K.) is also shown in FIG. 2. Surprisingly, the morphology of the cultured cancer cell in the cluster as indicated by an arrow in FIG. 1 and that of the original cancer cell of the patient's tumor as indicated by an arrow in FIG. 2 bears a close resemblance. As it is clear from Table 1, the composition ratio of the cancer cell to the fibroblast in the cultured cancer cell cluster can easily be controlled by regulating the bottom area of the cell culture dish, in other words, the fibroblast density in its confluent state ($6 \sim 8 \times 10^4$ cells/cm²) and the number of the seeded cancer cells. Furthermore, the size of the cancer cell cluster is dependent on the total cell number of the fibroblasts and cancer cells and it can be accurately controlled by the method of the present invention.

TABLE 1

| Size and Composition Ratio of Cancer Cell to Fibroblast in Cultured Cancer Cell Cluster | | | |
|---|---|---|---|
| Bottom Area of Dish (cm$^2$) | Concentration of Cancer Cell in Suspension (x 10$^4$/ml) | Composition Ratio of Cancer Cell (%) | Diameter of Cancer Cell Cluster (mm) |
| 1.9 | 2.0 | 8.3 | 0.7 |
| 1.9 | 10.0 | 40.2 | 0.8 |
| 3.8 | 2.0 | 4.9 | 1.2 |
| 3.8 | 10.0 | 21.3 | 1.3 |

Comparative Example 1

The 2 ml DMEM (containing 10% fetal calf serum) suspension (cell concentration: about 1.0 x 10$^5$ cells/ml) of the gastric cancer tumor cells prepared as in Example 1 was seeded onto a commercially available tissue culture dish (Falcon #3001, 35mm) and cultured in a 37°C carbon dioxide incubater (air/5% carbon dioxide). However, attachment and growth of the cancer cells did not take place at all.

Example 2

Ascites cancer cells metastasized from an original pancreatic cancer were isolated from 3.0 liters of abdominal ascites collected from a cancer patient (U.N., female, 63 years old) by centrifugation and suspended in the DMEM (containing 10% fetal calf serum).

The fibroblasts derived from human skin as used in Example 1 were seeded on a 12 well Cultureware coated with the same collagen and PNIPAAm mixture as employed in Example 1, and cultured for 5 days in a 37°C carbon dioxide incubater (air/5% carbon dioxide). As a result, a confluent monolayer of the fibroblasts was obtained. Then, 0.5 ml of the DMEM suspension of the above described cancer cells (cell concentration: about 8 x 10$^4$ cells/ml) was maintained at 37°C and seeded on to the confluent monolayer of the fibroblasts and left to stand in a 37°C carbon dioxide incubator (air/5% carbon dioxide) for 24 hours. After confirming that most of the cancer cells were attached on the monolayer by the phase contrast microscopic observance, the dish was taken out from the 37°C incubator and cooled down to 4°C. Under a phase contrast microscope it was observed that the cancer cell-attached monolayer of the fibroblasts gradually wrapped up the attached cancer cells in an engulfing fashion from the edge of the dish toward the center when it was detached as a cell sheet from the cell culture substrate. In about 5 minutes the cell sheet was completely released from the bottom of the dish and suspended in the culture medium. Separation of the cancer cells from the monolayer of the fibroblasts was not observed during the cell sheet detaching process. The cell sheet thus obtained was washed with a phosphate buffer solution twice to remove dissolved PNIPPAm and collagen and then transferred to the same cell non-adhesive dish as used in Example 1, and cultured in a 37°C carbon dioxide incubater (air/5% carbon dioxide). During the suspension culture, the cell sheet was gradually transformed into a cluster. After suspension culturing for 21 days, the cultured cancer cell cluster was fixed by a 10% formalin buffer solution and the paraffin-embedded tissue section was prepared. The cancer cell cluster section thus prepared was stained with hematoxylin-eosin and observed under a phase contrast microscope. The morphology of the cultured cancer cells in this cluster bears a close resemblance to that of the original pancreatic cancer cells.

Comparative Example 2

A 2 ml DMEM (containing 10% calf fetal serum) suspension (cell concentration: about 8 x 10$^5$ cells/ml) of the ascites cancer cells metastasized from the original pancreatic cancer as used in Example 2 was seeded on to a commercially available tissue culture dish (Falcon #3001, 35mm) and cultured in a 37°C carbon dioxide incubater (air/5% carbon dioxide). However, attachment and growth of the cancer cells did not take place at all.

Example 3

The pleural effluent of a patient (E.K., male, 68 years old) with cholangiocarcinoma was aseptically collected and the cancer cells in the pleural effluent were isolated from erythrocytes and lymphocytes by specific gravity centrifugation using Ficol. The above described primary cancer cells were suspended in RPMI 1640, seeded onto the 12 well Cultureware at a cell concentration of about $5 \times 10^4$ cell/well and cultured at 37°C in a carbon dioxide incubator (air/5% carbon dioxide) and the passages were repeated by trypsinization. By the repeated passage processes the cell line of cholangiocarcinoma was isolated and established.

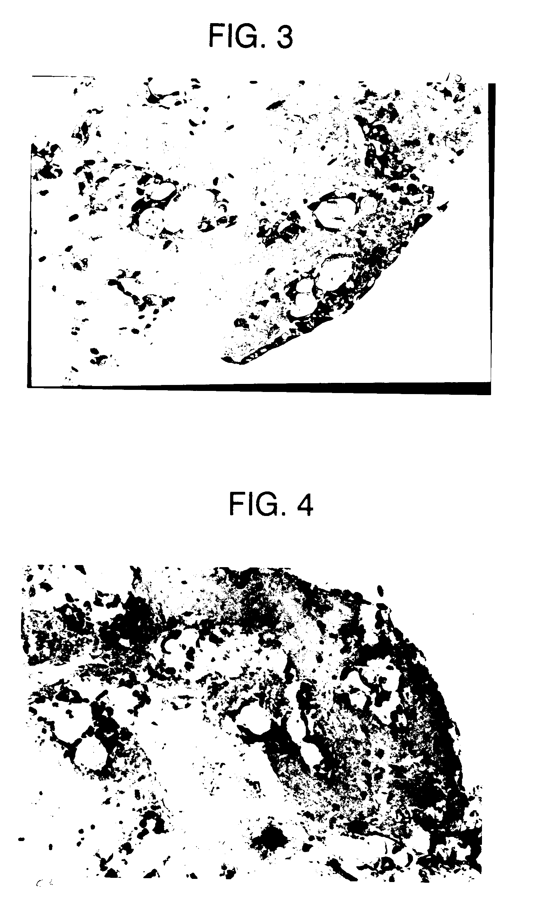

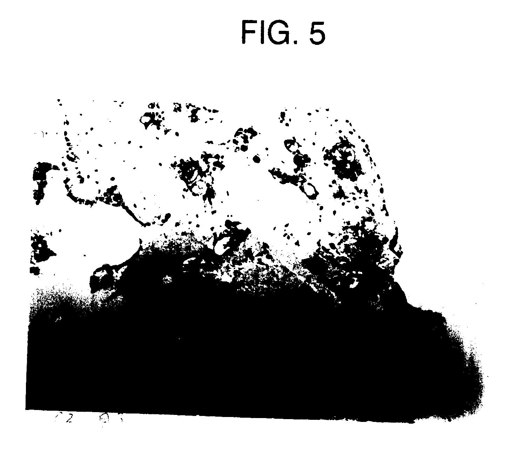

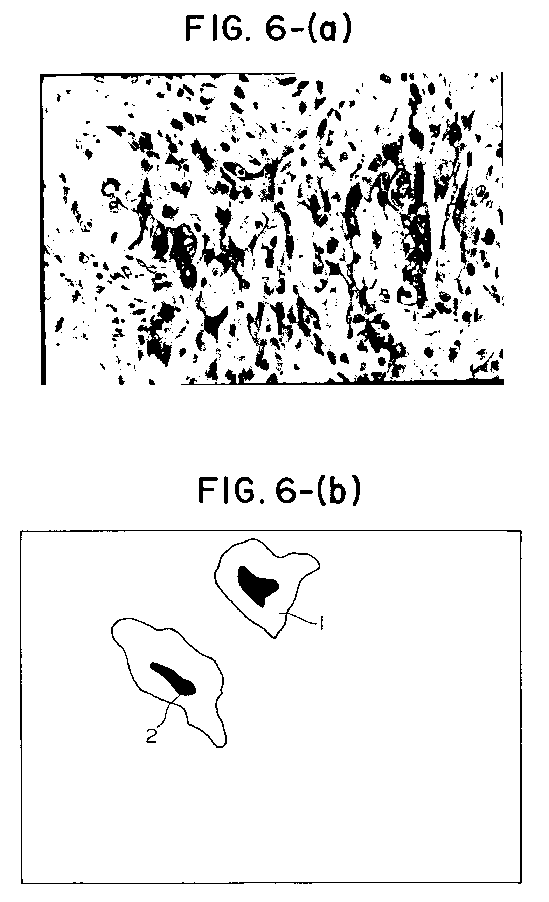

On the other hand, human dermal fibroblasts were used as the normal cells and 2 ml of the suspension of the fibroblasts in DMEM(containing 10% calf fetal serum) (cell concentration: about $2 \times 10^5$ cells/ml) was seeded onto a tissue culture dish (Falcon #3001, 35 mm) coated with the same collagen and PNIPAAm mixture as in Example 1 and cultured in a 37°C carbon dioxide incubator (air/5% carbon dioxide) for 5 days. After formation of a confluent monolayer of the fibroblasts, 0.5 ml of the suspension of the above described cancer cells in DMEM (cell concentration: about $2 \times 10^5$ cells/ml) was seeded onto the monolayer of the fibroblasts. The dish was left to stand at 37°C in a carbon dioxide incubator for 2 hours. As a result, the above described cancer cells were attached on the monolayer of the fibroblasts. Then the dish was then removed from the 37°C incubator and cooled down to 4°C. The cancer cell-attached monolayer of the fibroblasts was completely detached from the bottom of the dish as a cell sheet. When the detaching process of this monolayer was observed under a phase contrast microscope, it was found that the cell sheet was gradually detached from the edge of the dish toward its center, and it was also confirmed that the detached monolayer of the fibroblasts wrapped up the attached cancer cells in an engulfing fashion. Separation of the cancer cells from the monolayer layer was not observed during the detaching process of the cell sheet. The detachment was completed in about 5 minutes and the detached cell sheet was suspended in the culture medium. The cell sheet was washed with a phosphate buffer solution two to three times to remove dissolved PNIPPAm and collagen and then transferred to a cell non-adhesive dish (a dish coated with agar) and cultured in a 37°C carbon dioxide incubater (air/5% carbon dioxide). During suspension culture the cell sheet was transformed into a cell cluster in the cell non-adhesive dish. On the 7th day of suspension culture, the cultured cancer cell cluster was fixed by means of a 10% formalin buffer solution and a paraffin-embedded tissue section was prepared. The cancer cell cluster stained with hemotoxylin-eosin is shown in FIG. 3. As shown in FIG. 3, a pseudoglandular structure is clearly observed in this cancer cell cluster. In addition, in FIGS. 4 and 5, the cancer cell clusters immuno-stained with Carbohydrate Antigen 19-9 (CA.19-9, CIS Co., Ltd., France) and Carcino Embryonic Antigen (CEA, DAKO Co., Ltd., U. S. A.), respectively, are shown. Surprisingly the pseudoglandular structure was mainly formed by the positively immuno-stained cancer cells. For comparison, hematoxylin-eosin staining of the original tumor of the cancer patient (E.K.) is shown in FIG. 6(a) and the schematic drawing of the glandular structure of FIG. 6(a) is FIG. 6(b). The pseudoglandular structure in the cultured cancer cell cluster demonstrated in Figure 3 closely resembles the original glandular structure of the patient tumor demonstrated in FIGS. 6(a) and 6(b). In addition, from the evidence that the blood levels of CA.19-9 and CEA were very high in the patient, it is estimated that the original tumor tissue will be positive against CA.19-9 and CEA. This point also suggests that the cultured cancer cell cluster shown in FIGS. 4 and 5 functionally resembles the original tumor of the patient.

Example 4

Breast tumors were isolated from six patients (H.H., female, 51 years old; H.K., female, 40 years old; M.S., female, 70 years old; T.S., female, 50 years old; Y.Y., female, 50 years old; and M.S., female, 42 years old) under an aseptic condition during the surgical operations. Each tumor was placed in a Petri dish and cut into thin strips of about 1 mm$^3$. Then, the cancer tissue section was digested in a RPMI-1640 culture medium containing 10 mg/ml collagenase (type I, Sigma) at 37°C by stirring with a stirrer for about 60 minutes and the cancer cells were isolated by filtering the digested solution through a gauze, carrying out specific gravity centrifugation at 1500 r.p.m. for 30 minutes using Ficol and removing contaminated blood components. The entire amount of the primary breast cancer cells prepared from each of the tumor samples by the above described procedures as shown in Table 2 was suspended in 1 ml of RPMI-1640 culture medium containing 10% of fatal calf serum and seeded on a monolayer of human dermal fibroblasts formed in the same manner as in Example 3. After one day culture in a 37°C carbon dioxide incubator, the fibroblast monolayer attached with the cancer cells was detached as a cell sheet and a cancer cell cluster was prepared by culturing the cell sheet on a cell non-adhesive dish in the same manner as in Example 3. The culturing time of each cluster is also shown in Table 2. After culturing, each cluster was fixed, stained by hematoxylin-eosin and observed under a phase contrast microscope. As a result, in the cell clusters prepared from the tumors of the six patients the existence of the primary breast cancer cells was confirmed.

TABLE 2

| Entire Amount of Breast Cancer Cells Isolated from Six Patients and Culturing Time | | | |
|---|---|---|---|
| Run No. | Name of Patient | Entire Amount of Cancer Cells (cells) | Culturing Time (days) |
| 1 | H. H. | $10^4$ | 21 |
| 2 | H. K. | $8 \times 10^3$ | 34 |
| 3 | M. S. | $10^3$ | 27 |
| 4 | T. S. | $5 \times 10^4$ | 37 |
| 5 | Y. Y. | $10^5$ | 21 |
| 6 | M. S. | $1.5 \times 10^5$ | 14 and 28 |

**Claims**

1. A cancer cell cluster comprising cancer cells and normal cells having a controlled size and a controlled composition of said cancer cells and said normal cells.

2. The cancer cell cluster of Claim 1, wherein said cancer cell cluster has the pseudoglandular structure which is a characteristic feature of an adenocarcinoma.

3. The cancer cell cluster of Claim 1, wherein said cancer cells are primary cancer cells.

4. The cancer cell cluster of Claim 1, wherein said normal cells are fibroblasts.

5. A method for preparing a cancer cell cluster comprising normal cells and cancer cells which comprises the steps of:
   (i) seeding and culturing normal cells on a cell culture substrate comprising
      (a) a temperature-responsive polymeric compound having an LCST and
      (b) a cell adhesive substance, at a temperature higher than said LCST until said normal cells form a confluent monolayer of normal cells;
   (ii) seeding and attaching cancer cells on said monolayer of normal cells at a temperature higher than said LCST;
   (iii) detaching the monolayer of normal cells attached with said cancer cells from said cell culture substrate by lowering said temperature to a temperature below said LCST; and
   (iv) culturing the detached monolayer on a cell non-adhesive substrate to form a cancer cell cluster.

6. The method of Claim 5, wherein the composition of the cancer cells and the normal cells in said cell cluster is controlled by regulating the concentrations of each of said cancer cells and said normal cells seeded.

7. The method of Claim 5, wherein said cancer cells are primary cancer cells.

8. The method of Claim 5, wherein said normal cells are fibroblasts.

9. The method of Claim 5, wherein said temperature-responsive polymeric compound is selected from the group consisting of a poly-N-substituted acrylamide or a copolymer thereof, a poly-N-substituted methacrylamide derivative or a copolymer thereof, a polyvinyl methyl ether and a partially acetylated polyvinyl alcohol.

10. The method of Claim 9, wherein said poly-N-substituted acrylamide is poly(N-isopropyl acrylamide).

11. The method of Claim 5, wherein said cell adhesive substance is selected from the group consisting of collagen, fibronectin, laminin, vitoronectin, proteoglycan, glycosaminglycan, thrmobospondin, gelatin, lectins, anchorage oligopeptides, adhesive proteins isolated from shellfish, positively charged polymers and their mixtures.

**12.** The method of Claim 11, wherein said cell adhesive substance is collagen.

**13.** A cancer cell cluster prepared by the method of Claim 5.

**14.** A cancer cell cluster prepared by the method of Claim 6.

# FIG. 1

# FIG. 2

## FIG. 3

## FIG. 4

FIG. 5

# FIG. 6-(a)

# FIG. 6-(b)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | EP-A-0 455 319 (W.R. GRACE & CO.-CONN.)<br>* Whole document * | 1,5,9-13 | C12N5/00 |
| Y | EP-A-0 387 975 (W.R. GRACE & CO.-CONN.)<br>* Whole document * | 1-14 | |
| Y | EP-A-0 358 506 (MARROW-TECH INCORPORATED)<br>* Whole document, in particular page 11 lines 34 - 61 and page 36 example 17 * | 1-14 | |
| D,A | SCIENCE<br>vol. 240, no. 4849, 8 April 1988, WASHINGTON DC, US<br>pages 177 - 184;<br>R.M. SUTHERLAND: 'Cell and environment interactions in tumor microregions: the multicell spheroid model'<br>* Whole article * | 1-4, 13-14 | |
| Y | EP-A-0 382 214 (KAO CORPORATION ET AL.)<br>* Whole document * | 1-14 | **TECHNICAL FIELDS SEARCHED (Int. Cl.5 )** |
| Y | EP-A-0 328 839 (SANOFI S.A.)<br>* Whole document * | 1-14 | C12N |
| D,A | THE BRITISH JOURNAL OF CANCER<br>vol. 54, no. 4, October 1986, GB<br>pages 569 - 578;<br>E.K. ROFSTAD: 'Growth and radiosensitivity of malignant melanoma multicellular spheroids initiated directly from surgical specimens of tumours in man'<br>* Whole article * | 1-4, 13-14 | |
| Y | BIOTECHNOLOGY<br>vol. 8, September 1990, NEW YORK, US<br>pages 854 - 856;<br>T. TAKEZAWA ET AL.: 'Cell culture on a thermo-responsive polymer surface'<br>* Whole article * | 1-14 | |

The present search report has been drawn up for all claims

| Place of search | Date of completion of the search | Examiner |
|---|---|---|
| BERLIN | 08 MAY 1992 | JULIA P. |

EPO FORM 1503 03.82 (P0401)

European Patent
Office

**EUROPEAN SEARCH REPORT**

Application Number

EP    92 10 2162
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| | --- | | |
| Y | EP-A-0 278 817 (SANOFI S.A.) <br> * Whole document * | 1-14 | |
| | ----- | | |
| | | | |
| | | | TECHNICAL FIELDS SEARCHED (Int. Cl.5 ) |

The present search report has been drawn up for all claims

| Place of search | Date of completion of the search | Examiner |
|---|---|---|
| BERLIN | 08 MAY 1992 | JULIA P. |

**CATEGORY OF CITED DOCUMENTS**

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

........................................................................

& : member of the same patent family, corresponding
    document

EPO FORM 1503 03.82 (P0401)